# EUROPEAN PATENT APPLICATION

(11) **EP 3 460 471 A1**
(43) Date of publication of application: **27.03.2019**
(21) Application number: 18194226.9
(22) Date of filing: 13.09.2018
(51) Int. Cl.: G01N 33/50, G01N 15/14

(54) **CELL ANALYSIS METHOD, CELL INFORMATION PROVIDING APPARATUS, CELL INFORMATION PROVIDING SYSTEM, AND RECORDING MEDIUM**

(30) Priority: 20.09.2017 JP 2017180304
(71) Applicant: SYSMEX CORPORATION, Kobe-shi Hyogo 651-0073 (JP)
(72) Inventor: Inokuchi, Hiroaki, Hyogo, 651-0073 (JP); Shirasuna, Kei, Hyogo, 651-0073 (JP)
(74) Representative: Hoffmann Eitle

(57) **Abstract**

The method includes a counting step of counting, among cells included in a sample, a first cell number indicating the number of cells classified into a first group based on cell size and cell nucleus size and having a DNA amount greater than a threshold value, and a second cell number indicating the number of cells classified into a second group that is different from the first group based on the cell size and cell nucleus size and having a DNA amount greater than the threshold value; and a comparing step of comparing the first cell number with the second cell number.

## Description

### FIELD OF THE INVENTION

The present invention relates to a cell analysis method, a cell information providing apparatus, a cell information providing system and the like.

### BACKGROUND

Analytical apparatuses that automatically analyze cells collected from epithelial tissues of a subject and provide information on canceration of epithelial tissues are known (for example, see U.S. Patent Application Publication No. 2014/199702). Epithelial tissues such as the cervix and oral cavity are formed from basal layer, parabasal layer, intermediate layer, and surface layer. The analyzer described in U.S. Patent Application Publication No. 2014/199702 acquires measurement data including data showing the size of the cell, data indicating the size of the cell nucleus, and data indicating the amount of DNA possessed by the cell relative to cells collected form a subject, and specifies the measurement data of cells close to the basal membrane as the analysis target based on the N/C ratio which is the ratio of the cell nucleus size to the cell size. This analyzer calculates the ratio of the number of cells having the G0 stage or G1 stage DNA amount to the number of cells having the S stage or more DNA amount based on the DNA amount data contained in the specified measurement data, and determines whether re-examination is required (positive) based on whether the calculated ratio exceeds a threshold.

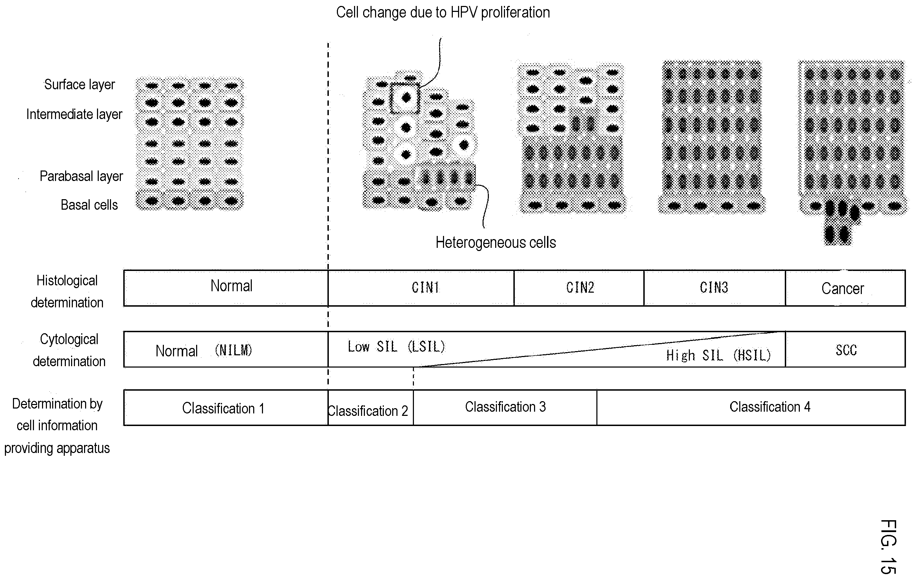

For example, in histological diagnosis of the cervix, as shown in "determination of histological diagnosis" in FIG. 15, the process from a normal state to the cancerous state is divided into several stages of "normal", "CIN 1" , "CIN 2", "CIN 3", and "cancer". In the analyzer described in U.S. Patent Application Publication No. 2014/199702, measurement data of cells on the basal membrane side where cells that are heterogeneous at the early stages of cancer are likely to appear are analyzed. Therefore, it is possible to distinguish whether the subject is in an early stage of cancer such as "CIN 2" to "CIN 3", or in the pre-cancer stage "CIN 1" determined to not require treatment.

However, it is desirable to be able to grasp the state of the cell of the subject in more detail, for example, regarding the degree of possibility that the phase will shift to "CIN 2" in the future if it is in the stage before "CIN 1", and degree of progress of possibility of canceration when in the stage after "CIN 2".

### SUMMARY OF THE INVENTION

In order to solve the above problems, the cell analysis method according to one aspect of the invention includes a counting step of counting, among cells included in a sample, a first cell number indicating the number of cells classified into a first group based on cell size and cell nucleus size and having a DNA amount greater than a threshold value, and a second cell number indicating the number of cells classified into a second group that is different from the first group based on the cell size and cell nucleus size and having a DNA amount greater than the threshold value; and a comparing step of comparing the first cell number with the second cell number.

According to the above configuration, it is possible to acquire information indicating the state of existence of cells with a high DNA amount in each of different groups classified on the basis of cell size and cell nucleus size, and it is possible to provide information for grasping the state of the cells of the subject in more detail, such as the state of development of cancer.

In the counting step, the cells included in the sample also may be classified based on the size of the cells and the size of the cell nucleus so that the cells on the surface side are included in the first group and the cells on the basal side are included in the second group.

In this way it is possible to acquire information indicating the state of existence of cells having a large amount of DNA in the surface side and the base side, respectively, and information for a more detailed comprehension of the cellular condition of the subject can be provided taking into consideration not only the state of the base side but also the state of the surface layer side.

In the counting step, the cells included in the sample also may be classified into the first group and the second group based on the ratio between the size of the cell and the size of the cell nucleus.

In the counting step, the cells also may be classified into the first group and the second group by comparing the ratio with a second threshold value.

In the counting step, cells having a DNA amount higher than the threshold value also may be counted based on the amount of fluorescence obtained from the cell nucleus. Note that the amount of fluorescence is fluorescence information that reflects the amount of DNA in the cell nucleus, and includes the pulse area, peak intensity, and the like of the signal waveform of fluorescence obtained from the cell nucleus.

The threshold value may be a value set between a value indicating a DNA amount of a diploid cell and a value indicating a DNA amount larger than a diploid cell.

The cells contained in the sample also may be cells detached from the tissue.

The tissue may be an epithelial tissue.

In an outputting step, information on the presence or absence of dysplasia in the surface layer and the intermediate layer of the tissue may be output depending on whether the first cell number is larger than the second cell number.

A second counting step in which a third cell number indicating the number of cells in which the amount of DNA contained in the cells is equal to or lower than the threshold value among cells included in the sample and a fourth cell number indicating the number of cells having DNA amounts larger than the threshold value also may be provided, so that the state of the tissue based on a result of comparison between the first cell number and the second cell number and a result of comparison result between the third cell number and the fourth cell number can be output in on the basis thereof in the outputting step.

According to the above configuration, it is possible to further improve the reliability of the information to be output by using information regarding whether the number of cells having a larger DNA amount than the diploid cells among the cells of the epithelial tissue is large or small.

In order to solve the above problems, the cell analysis method according to one aspect of the present invention is a method for analyzing cells contained in a sample, include: a data acquiring step of acquiring first data indicating the size of a cell, acquiring second data indicating a size of a cell nucleus, and acquiring third data indicating a DNA amount of a cell based on the first data and the second data; a counting step of respectively counting a first cell number indicating the number of cells identified based on the first data among cells classified in a first group based on the first data and the second data, and counting a second cell number indicating the number of cells identified based on the third data among cells classified in the second group which is different from the first group based on the third data, and a comparing step of comparing the first cell number and the second cell number. According to the above configuration, the same effect as above-mentioned cell analysis method can be obtained.

In the counting step, cells contained in the sample also may be classified into the first group and the second group based on the ratio of the cell size and the cell nucleus size obtained from the first data and the second data.

In the counting step, the cells included in the sample also may be classified into the first group and the second group by comparing the ratio of the size of the cell and the size of the cell nucleus to a threshold value.

The threshold value also may be set between the ratio of the size of the intermediate layer cells to the size of the cell nucleus of the intermediate layer cells, and the ratio of the size of the parabasal layer cells and the size of the cell nucleus of the parabasal layer cells.

The first data also may be the pulse width of a scattered light signal generated from the cell, the second data may be the pulse width of a fluorescence signal generated from a cell nucleus of the cell, and the third data may be the pulse area of the fluorescence signal.

In order to solve the above problems, the cell information providing apparatus according to one aspect of the present invention includes a counting unit configured to count a first cell number indicating the number of cells having DNA above a threshold value by classifying a first group based on cell size and cell nucleus size among cells included in a sample, and counting a second cell number indicating the number of cells having an amount of DNA greater than the threshold by classifying a second group that is different from the first group based on the cell size and cell nucleus size, and a comparing unit configured to compare the first cell number and the second cell number. According to the above configuration, the same effect as above-mentioned cell analysis method can be obtained.

In order to solve the above problems, the cell information providing apparatus according to one aspect of the present invention includes a data acquiring unit configured to acquire first data indicating the size of a cell, acquire second data indicating a size of a cell nucleus, and acquire third data indicating a DNA amount of a cell based on the first data and the second data; a counting unit configured to respectively count a first cell number indicating the number of cells identified based on the first data among cells classified in a first group based on the first data and the second data, and count a second cell number indicating the number of cells identified based on the third data among cells classified in the second group which is different from the first group based on the third data, and a comparing unit configured to compare the first cell number and the second cell number. According to the above configuration, the same effect as above-mentioned cell analysis method can be obtained.

A determining unit that determines the state of the tissue based on a comparison result by the comparing unit also may be provided.

A second counting unit configured to count a third cell number indicating the number of cells in which the amount of DNA contained in the cells is equal to or lower than the threshold value among cells included in the sample and a fourth cell number indicating the number of cells having DNA amounts larger than the threshold value also may be provided, so that the determining unit can determine the state of the tissue based on a result of comparison between the first cell number and the second cell number and a result of comparison result between the third cell number and the fourth cell number. By providing such a configuration, it is possible to further improve the reliability of the determination result.

The data acquiring unit also may acquire the first data, the second data, and the third data from a measurement apparatus including a flow cytometer.

Since the measuring apparatus includes a flow cytometer, the measurement apparatus can output first data showing the size of cells contained in the sample, the second data showing the size of the cell nucleus of the cell, and third data showing the amount of DNA possessed by the cell. Therefore, the cell information providing apparatus can utilize suitable measurement data obtainable from the flow cytometer.

In order to solve the above problems, a cell information providing system according to one aspect of the present invention includes the cell information providing apparatus, a storage device for storing a determination result by the cell information providing apparatus in association with identification information of the sample, and a microscopy terminal apparatus for acquiring an image obtained by imaging a sample having identification information identical to the identification information of the sample, reading the determination result corresponding to the identification information from the storage device, and displaying the image and the determination result.

According to the above configuration, effects similar to those of the cell analysis method and the cell information providing apparatus are obtained. For example, if information on cellular dysplasia of tissue is provided to a cytodiagnostician who performs pathological diagnosis such as cytodiagnosis and histological diagnosis, the pathologic diagnosis can be efficiently and appropriately performed, and the reliability of the diagnostic result is improved.

The cell information providing apparatus according to each aspect of the present invention may be realized by a computer, in which case a control program of the cell information providing apparatus and a computer readable medium storing the control program causes the computer to realize the cell information providing apparatus by operating the computer as each part (software element) provided in the cell information providing apparatus.

According to one aspect of the invention, it is possible to provide information for comprehending the state of a cell of a subject in more detail.

### BRIEF DESCRIPTION OF THE DRAWINGS

FIG. 1 is a diagram showing a structural example of a cell information providing system according to an embodiment of the invention;
FIG. 2 is a schematic diagram describing cell layers constituting epithelial tissue;
FIG. 3 is a diagram describing the relationship between a histogram of DNA amount and the cell cycle;
FIG. 4 is a block diagram showing a structural example of a measuring apparatus;
FIG. 5 is a block diagram showing a structural example of an optical detection unit and an imaging unit provided in a measurement apparatus;
FIG. 6 is a diagram illustrating a method of calculating various data from measurement data measured by a measuring apparatus;
FIG. 7 is a block diagram illustrating a configuration of a computer usable as a cell information providing apparatus;
FIG. 8 is a block diagram showing a structural example of a cell information providing apparatus according to an embodiment of the invention;
FIG. 9 is a flowchart showing an example of a processing flow performed by a cell information providing apparatus;
FIG. 10 is a schematic diagram illustrating the number of cells counted by a first counting unit;
FIG. 11 is a flowchart showing another example of a processing flow performed by a cell information providing apparatus;
FIG. 12 is a schematic diagram illustrating the number of cells counted by a second counting unit;
FIG. 13 is a diagram showing an example of a classification table;
FIG. 14 is a diagram showing an example of a comment correspondence table;
FIG. 15 is a diagram illustrating the relationship between precancerous lesions in epithelial tissue and classification in cell diagnosis;
FIG. 16 is a diagram showing an example of a screen display on which a microscopy terminal device displays a microscope image and a determination result by a cell information providing device; and
FIG. 17A and FIG. 17B are a result of an embodiment in which a determination result by a cell information providing apparatus is verified.

### DESCRIPTION OF THE EMBODIMENTS OF THE INVENTION

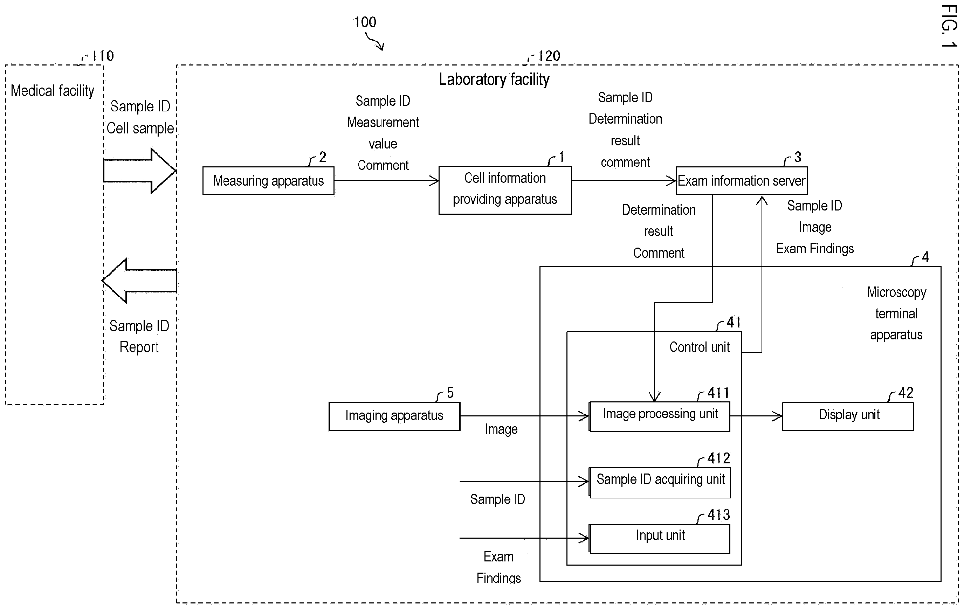

A schematic configuration of a cell information providing system 100 using a cell information providing apparatus 1 according to one embodiment of the invention will be described below with reference to FIG. 1. FIG. 1 is a diagram showing a structural example of a laboratory facility 120 that utilizes a cell information providing system 100 according to an embodiment of the invention. As shown in FIG. 1, the cell information providing system 100 is a system suitably applicable to the laboratory facility 120 which analyzes cells contained in a cell sample provided from a medical institution 110.

### Medical Facility 110

The medical facility 110 shown in FIG. 1 is a facility for examining a subject such as a patient, and cells of the subject can be collected as necessary. In medical facility 110, necessary pretreatment for analysis and examination may be performed by the laboratory facility 120; in this case, the pretreated cells are provided to the laboratory facility 120 as cell samples. Note that a sample ID is given to each cell sample and is associated with information of the subject from which the cell originated.

In the present specification, the case of analyzing the cells of epithelial tissue, such as the uterine cervix will be described as an example. Note that, in the cell information providing system 100 according to one embodiment of the invention, cells other than the uterine cervix include cells detached from epithelial tissue from, for example the oral cavity, esophagus, bronchus and the like. Cells detached from the endometrium, cells in the urine separated from the urinary organ, and cells in the cavity fluid can also be analyzed by the cell information providing system 100.

### Composition of Epithelial Tissue and Characteristics of Cells Included in Epithelial Tissue

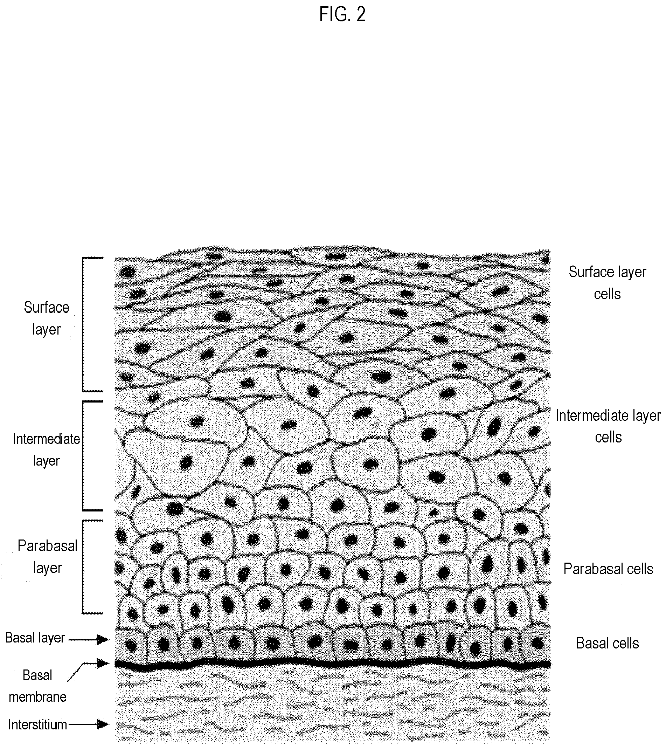

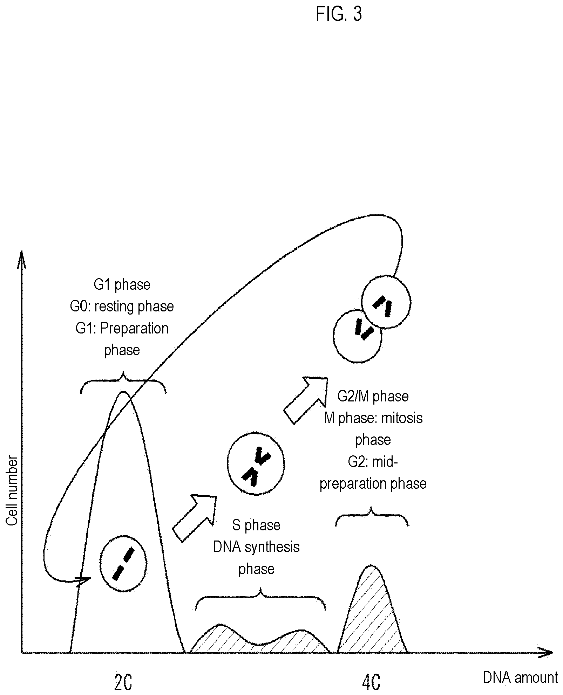

The composition of the epithelial tissue and the characteristics of the cells contained in the epithelial tissue will be described below with reference to FIGS. 2 and 3. First, the composition of the epithelial tissue to be determined by the cell information providing apparatus 1 and a summary of the features of the cells contained in the epithelial tissue will be described with reference to FIG. 2. FIG. 2 is a schematic diagram illustrating the cell layers constituting epithelial tissue.

In the epithelium of these tissues, plural types of cells exist in layer in sequence from the basal membrane. Note that in this specification the surface layer and the intermediate layer located in the upper layer are referred to as the surface layer side when the basal membrane is the lower layer. On the other hand, the parabasal layer and basal layer are referred to as the basal layer side or deep layer system. For example, in the cervix and oral mucosa, the side adjacent to the exterior world corresponds to the surface layer side.

In the cervix, from the basal membrane side, a layer (basal layer) formed by basal cells, a layer (parabasal layer)formed by parabasal cells, a layer (intermediate layer) formed by intermediate layer cell, and a layer (surface layer) formed by surface layer cells are formed, as shown in FIG. 2. Basal cells near the basal membrane are differentiated to parabasal cells, parabasal cells to intermediate layer cells, and intermediate layer cells to surface cells. In the oral mucosa, a layer of basal cells, a layer of spinous cells, a layer of granular cells, and a stratum corneum are formed in sequence from the basal membrane side. These are summarized in Table 1 below.

**Table 1-1**

| | Cell name | | N/C ratio | |
|---|---|---|---|---|
| | Cervical squamous cells | Oral mucosal cells | Cervical squamous cells | Oral mucosal cells |
| Surface layer side | | Stratum corneum | | |
| | | | | |
| | Surface layer cells | Granular cells | Low | Low |
| | | | | |
| | Intermediate layer cells | Spinous cells | | |
| | Parabasal cells | | | |
| | | | | |
| Basal membrane side | Basal cells | Basal cells | High | High |

Of the plurality of types of cells constituting the epithelium, the cells involved in carcinogenesis are basal cells. In the process leading to cancer, the basal cells are heterogeneous and become heterotypic cells (cells that acquire dysplasia and become heterotypic cells). Heterotypic cells acquire proliferative capacity and occupy from the basal layer side to the surface layer side. Therefore, in the early stages of cancer, there are many cancerous cells among the cells existing in the basal, parabasal, and intermediate layers of the epithelial tissue of the cervix. In the case of the epithelial tissue of the oral mucosa, there are many cancerous cells among the cells existing in the basal cell layer and the spinous cell layer. Conversely, in early stages of cancer, cells present in the surface layer side of the epithelium such as the cervical epithelial tissue and the stratum corneum of the epithelial tissue of the oral mucosa are rarely cancerous.

In the epithelial tissue described above, it also is known that the cell size gradually decreases from the layer on the surface layer side to the layer on the basal membrane side, and it is understood that the size of the cell nucleus gradually increases. Therefore, the ratio of the size of the cell nucleus to the size of the cell (the N/C ratio to be described later) also gradually increases from the layer on the surface layer side to the layer on the side of the basal membrane. The morphology of each cell constituting the epithelial tissue of the cervix, the size of the cytoplasm (that is, the size of the cell), and the size of the cell nucleus are shown in Table 2 below.

**Table 2**

| Name | Cell morphology | Cell size | Nucleus size |
|---|---|---|---|
| Surface cells | Polygonal form | 50-60 *µ*m | 5 *µ*m |
| Intermediate cells | Polygonal∼Oval | 30-50 *µ*m | 8 *µ*m |
| Parabasal cells | Oval | 20 *µ*m | 9 *µ*m |
| Basal cells | Circular∼Oval | 12-14 *µ*m | 8-10 *µ*m |

According to Table 2, in the case of the epithelial tissue of the cervix, the N/C ratio of basal cells is 0.57 to 0.83, the N/C ratio of parabasal cells is 0.45, the N/C of intermediate layer cells is 0.16 to 0.27, and the N/C ratio of the surface layer cell is 0.08 to 0.1. Therefore, for example, cells having an N/C ratio of 0.3 or more are deep layer cells including parabasal cells and basal cells, and cells having an N/C ratio of less than 0.3 are surface/intermediate layer cells. In this way, in tissues where squamous metaplasia occurs, such as the cervix, oral cavity, esophagus, or bronchus, it is possible to distinguish whether the cell is a deep system cell or a surface/intermediate layer cell based on the value of N/C ratio.

The change in the DNA amount of the cells and the DNA amount of the heterozygous cells will be described next with reference to FIG. 3. FIG. 3 is a diagram illustrating the relationship between a histogram of DNA amount and cell cycle.

As shown in FIG. 3, a cell divides and propagates into two cells via events such as DNA replication, chromosome partitioning, nuclear division, cytokinesis and the like according to a fixed cycle (cell cycle). The divided cells return to the starting point. The cell cycle can be divided into the following 4 phases depending on the stage (stage).
G1 phase: the period of preparation and inspection for entering the S phase.
S phase: DNA synthesis phase.
G2 phase: the period of preparation and inspection for entering the M phase.
M phase: mitosis phase.

That is, the cells are in one of five stages which includes a quiescent period (G0 phase) where cell propagation is paused in addition to the four phases described above.

In the process of propagating according to the cell cycle, the chromosome of the cell nucleus also grow. Therefore, it can be estimated at which stage of the cell cycle the cell is by measuring the amount of DNA in a cell. In the case of normal cells, the amount of DNA in the G1 phase is constant, the amount of DNA gradually increases in the subsequent S phase, then becomes a constant value in the G2 phase, and this value is also maintained in the M phase. An example of a histogram of the amount of DNA relating to cells collected from tissues such as epithelium composed of normal cells is shown in FIG. 3. The peak with the highest peak corresponds to a cell in the G0 or G1 phase with the lowest amount of DNA, and the peak with the next highest peak corresponds to a cell in the G2 or M phase with the highest amount of DNA, and intermediate to these corresponds to cells in the S phase.

When the cells to be analyzed are all normal cells, the ratio of the number of cells in any stage of the S phase, G2 phase, or M phase to the number of cells in G0 phase or G1 phase is a value within a substantially constant range. However, when cancerous cells or heterozygous cells in the course of canceration are included, there is an increase in the existence frequency of abnormal cells with abnormally high numbers of chromosomes and a large amount of DNA compared to normal cells.

For example, when estimating whether the cells to be analyzed contain cancerous cells or heterozygous cells in the course of canceration, the ratio of the number of cells having a DNA amount larger than the DNA amount possessed by cells in the normal G0 or G1 phase relative to the number of normal G0 or G1 cells may be used as a criterion. Specifically, in the DNA amount histogram shown in FIG. 3, the leftmost peak corresponds to a cell having a DNA amount corresponding to the DNA amount possessed by normal cells in the G0 or G1 phases, and the three peaks on the right side correspond to cells having a DNA amount higher than the DNA amount of normal cells in the G0 or G1 phases. A cell having a DNA amount equivalent to that of normal cells in the G0 or G1 phases will be referred to as "cells having an amount of DNA corresponding to diploid cells" below. A cell having a DNA amount larger than the DNA amount possessed by the normal cells in the G0 or G1 phases is referred to as "a cell having a larger amount of DNA than the diploid cell" or the like. In FIG. 3, the range showing the normal amount of DNA is denoted as "2C", and the range showing the DNA amount possessed by the cells in G2 phase and M phase is denoted as "4C".

These three peaks on the right side correspond to cells in the S phase (the two peaks in the middle) shown in FIG. 3, or cells in the G2 / M phase (the rightmost peak), and may be considered cancerous cells and heterozygous cells in the process of canceration. As the number of cancer cells increases, the three peaks on the right side are considered to become larger. Note that although FIG. 3 shows an example in which there are three peaks for the sake of clarity, the number that can be recognized as a peak depends on the state of the subject when preparing a DNA amount histogram of the cells contained in the epithelial tissue of the uterine cervix actually collected from the subject.

### Laboratory Facility 120

Returning to FIG. 1, the laboratory facility 120 analyzes and examines a cell sample delivered from the medical facility 110. Although not limited thereto, the laboratory facility 120 is provided with a measuring apparatus 2, a cell information providing apparatus 1, an exam information server 3, a microscopy terminal apparatus 4 and the like. In the laboratory facility 120, the cell sample is analyzed and examined according to the analysis request from the medical facility 110, and a report is prepared based on the analysis result and the examination result. The report is provided to the medical facility 110 as the analysis requesting source.

### Structure of Cell Information Providing System 100

The structure of the cell information providing system 100 provided in the laboratory facility 120 will be described hereinafter. As shown in FIG. 1, the cell information providing system 100 includes a measuring apparatus 2, a cell information providing apparatus 1, an exam information server 3, and a microscopy terminal apparatus 4 used by a cytodiagnostician for cytodiagnosis and histological diagnosis of a cell sample. Details of the configurations of the measuring apparatus 2, the cell information providing apparatus 1, and the microscopy terminal apparatus 4 will be described later.

The exam information server 3 stores the determination results obtained by the cell information providing apparatus 1 in association with a sample ID (identification information) unique to the analysis sample. The microscopy terminal apparatus 4 acquires an image of the smear sample (sample) to which the sample ID is given, reads the determination result associated with the same sample ID as the smear sample from the exam information server 3, and both the image and the determination result are displayed on a display unit 42. Note that the same ID also may be the ID (for example, patient number and the like) assigned to each subject.

In the medical facility 110, cell samples prepared using the cells to be analyzed (collected) from the tissues of the subject are delivered to the laboratory facility 120 in a preprocessed state. The cell sample reaching the laboratory facility 120 may include a measurement sample (sample) to be supplied to the measuring apparatus 2, and a smear sample to be subjected to cytodiagnosis and histological diagnosis such as microscopy or the like. A unique sample ID is assigned to the cell sample, and the results of all the examinations performed in the laboratory facility 120, the result of the analysis, findings and the like are associated with the sample ID and stored in the exam information server 3.

That is, in the laboratory facility 120, captured images associated with each sample ID, exam findings and the like are read out from the exam information server 3, and a report is prepared using these data. The created report is provided to the medical facility 110 in association with the sample ID.

Generally, since the analysis by the measuring apparatus 2 is performed before the cytodiagnosis of the smear sample, the determination result from the cell information providing apparatus 1 is provided before the cytodiagnosis of the smear sample given the same sample ID. If such a system is applied, a person who performs cytodiagnosis using the microscopy terminal apparatus 4 refers to the determination result and comments on the cell sample provided from the cell information providing apparatus 1, and can then perform an appropriate a microscopic examination efficiently.

### Configuration of Measuring Apparatus 2

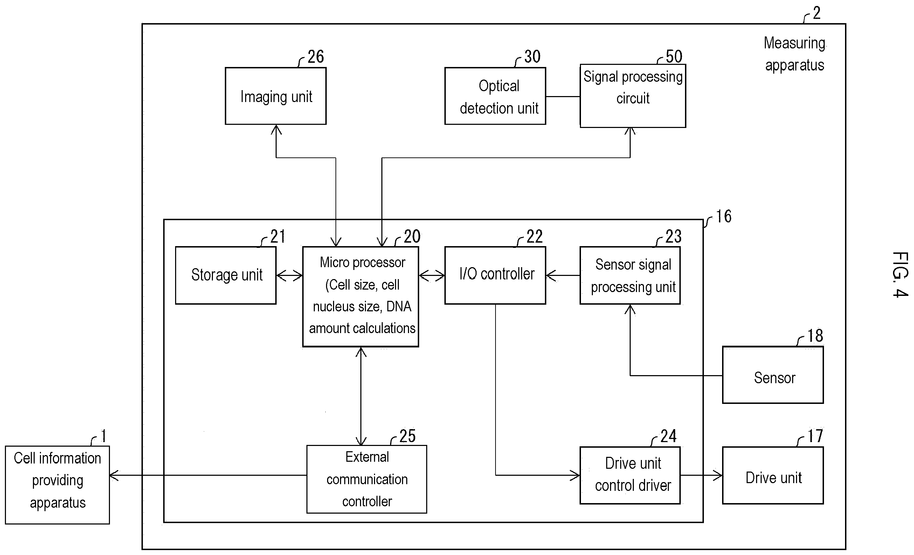

The structure of the measuring apparatus 2 will be described with reference to FIG. 4 below. FIG. 4 is a block diagram showing a structural example of the measuring apparatus 2.

As shown in FIG. 4, the measuring apparatus 2 provides measurement data to the cell information providing apparatus 1. The measuring apparatus 2 includes an optical detection unit 30 including a flow cytometer that detects forward scattered light signal waveform data FS and fluorescence signal waveform data FL and the like from cells included in the measurement sample, a signal processing circuit 50, a measurement control unit 16, a drive unit 17 such as a motor, an actuator, a valve and the like, a sensor 18 including various sensors, and an imaging unit 26 that captures images of cells.

The signal processing circuit 50 includes an analog signal processing circuit for performing amplification processing, filter processing and the like on the output of the optical detection unit 30 which has been amplified by a preamplifier (not shown), an A/D converter for converting the output of the analog signal processing circuit to digital signals, and a digital signal processing circuit for performing predetermined waveform processing on the digital signals. The measurement control unit 16 also controls the operation of the driving unit 17 while processing the signal of the sensor 18, so that the measurement sample is suctioned or measured.

A sample prepared by centrifuging, diluting (suspending), stirring, PI staining or the like performed on the cells of epithelial tissue of a subject (for example, cells collected from the uterine cervix) can be used as the measurement sample. The measurement sample prepared in this way is given a sample ID, placed in a predetermined position of a sample setting unit (not shown) while accommodated in a test tube, and then placed under a pipette (not shown) of the measuring apparatus 2, then is suctioned by the pipette and supplied to the flow cell together with a sheath liquid to form a sample flow in the flow cell. PI staining is a process of staining DNA using propidium iodide (PI) which is a fluorescent dye that binds to DNA by intercalating into a double helix of DNA. Since PI staining selectively stains cell nuclei, red fluorescence from the cell nucleus can be detected. PI staining is performed using a fluorescent dye solution containing a red pigment.

The measurement control unit 16 includes a microprocessor 20, a storage unit 21, an I/O controller 22, a sensor signal processing unit 23, a drive unit control driver 24, an external communication controller 25 and the like. The storage unit 21 includes a ROM, a RAM and the like, and the ROM stores a control program for controlling the driving unit 17 and data necessary for executing the control program. The microprocessor 20 can load the control program into the RAM or directly from the ROM.

A signal from the sensor 18 is transmitted through the sensor signal processing unit 23 and the I/O controller 22 to the microprocessor 20. By executing the control program, the microprocessor 20 can control the drive unit 17 via the I/O controller 22 and the drive unit control driver 24 in accordance with a signal from the sensor 18.

The microprocessor 20 calculates cell size (first data), cell nucleus size (second data), and DNA amount (third data) for each cell using measurement data such as FS and FL. The first data, the second data, and the third data are transmitted to an external device such as the cell information providing apparatus 1 via an external communication controller 25.

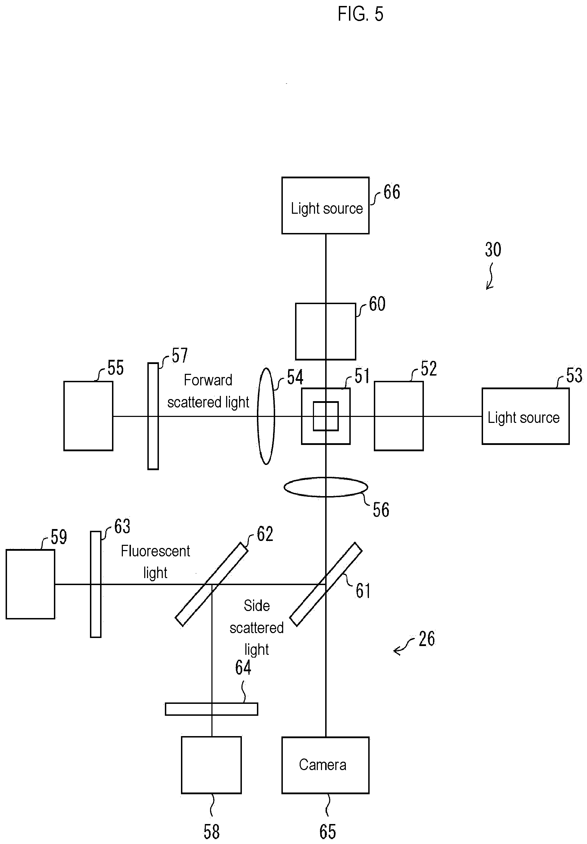

The structure of the optical detection unit 30 and the imaging unit 26 will be described below with reference to FIG. 5. FIG. 5 is a block diagram showing a structural example of the optical detection unit 30 and the imaging unit 26 included in the measurement apparatus. In the example shown here, the optical detection unit 30 comprises a flow cytometer and a light source 53 configured by a semiconductor laser, and the laser light emitted from the light source 53 passes through a lens system 52 to the measurement sample flowing through the flow cell 51. The forward scattered light generated from the cells in the measurement sample by the laser light is detected by a photodiode (light receiving unit) 55 via an objective lens 54 and a filter 57. Note that the lens system 52 is configured by a lens group including a collimator lens, a cylinder lens, a condenser lens and the like.

The fluorescence and the side scattered light generated from the cell also enter the dichroic mirror 61 via the objective lens 56 disposed on the side of the flow cell 51. Then, the fluorescence and the side scattered light reflected by the dichroic mirror 61 are incident on the dichroic mirror 62.

The fluorescence transmitted through the dichroic mirror 62 is detected by the photomultiplier 59 via the filter 63. The side scattered light reflected by the dichroic mirror 62 is detected by the photomultiplier 58 via the filter 64.

The photodiode 55, the photomultiplier 58, and the photomultiplier 59 convert the detected light into electric signals and output a forward scattered light signal (FSC), a side scattered light signal (SSC) and a fluorescence signal (SFC). These signals are amplified by a preamplifier (not shown), and then sent to the aforementioned signal processing circuit 50 (see FIG. 4).

As shown in FIG. 4, the forward scattered light waveform data (FS), the side scattered light waveform data (SS), and the fluorescent light waveform data (FL) obtained by performing signal processing such as filter processing and A/D conversion processing in the signal processing circuit 50 is used to calculate the first data, the second data, and the third data in the microprocessor 20. The calculated first data, second data, and third data are sent to the cell information providing apparatus 1 via the external communication controller 25.

Note that a gas laser may be used instead of the semiconductor laser as the light source 53, but it is preferable to employ a semiconductor laser from the viewpoint of low cost, small size, and low power consumption. By adopting the semiconductor laser, it is possible to reduce the product cost and to achieve downsizing and power saving of the apparatus. In the present embodiment, a blue semiconductor laser having a short wavelength which is advantageous for narrowing the beam is used. The blue semiconductor laser is also effective for a fluorescence excitation wavelength such as PI. Among the semiconductor lasers, a red semiconductor laser that is low in cost and long in life and stable in supply from manufacturers may be used.

In addition to the optical detection unit 30, the measurement apparatus 2 shown in FIG. 4 is provided with an imaging unit 26. As shown in FIG. 5, the imaging unit 26 includes a light source 66 configured by a pulsed laser and a CCD camera 65; the laser light from the light source 66 enters the flow cell 51 via the lens system 60, and further passes through an objective lens 56 and the dichroic mirror 61 to form an image on the CCD camera 65. The light source 66 emits light at a predetermined timing to enable imaging by the CCD camera 65.

As shown in FIG. 4, the image of the cell imaged by the CCD camera 65 may be sent to the cell information providing apparatus 1 via the external communication controller 25 by the microprocessor 20. In this case, the image of the cell is stored in the storage unit 12 in the cell information providing apparatus 1 in association with the first data, the second data, and the third data related to the cell.

### Method of Calculating First to Third Data

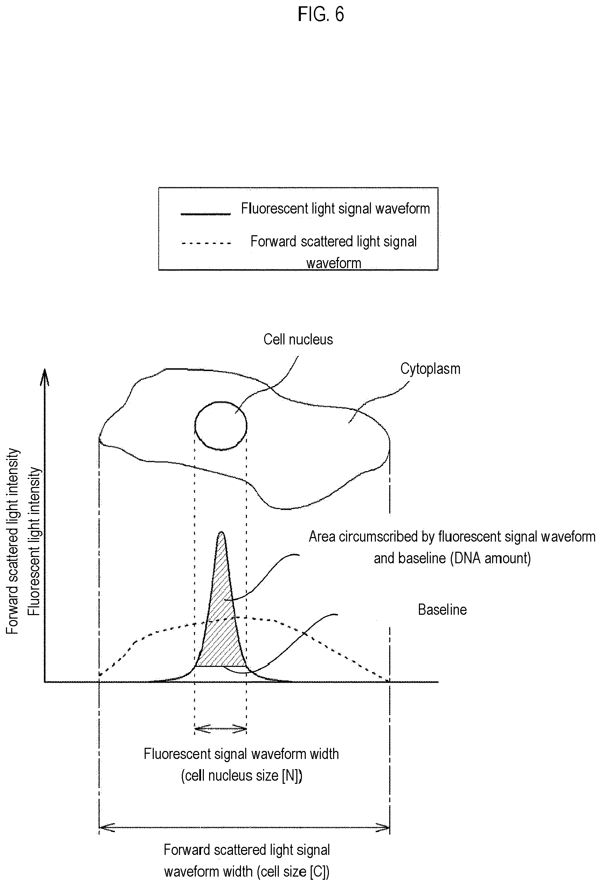

A method of calculating the first data, the second data, and the third data by the microprocessor 20 of the measuring apparatus 2 will be described below with reference to FIG. 6. FIG. 6 is a diagram illustrating a method of calculating various data from measurement data measured by the measuring apparatus 2.

FIG. 6 shows a schematic diagram of the cell containing the cytoplasm and the cell nucleus, and the forward scattered light signal waveform and the fluorescence signal waveform obtained from this cell. In FIG. 6, the vertical axis of the graph represents the intensity of forward scattered light and fluorescence. The width of the forward scattered light signal waveform represents the width of the cytoplasm, that is, the value indicating the cell size (C), and the width of the fluorescence signal waveform represents the value indicating the size (N) of the cell nucleus.

Based on the width of the forward scattered light signal waveform (the pulse width of the forward scattered light signal) generated from each cell and the width of the fluorescence signal waveform generated from the cell nucleus of each cell (pulse width of the fluorescence signal), the microprocessor 20 calculates the first data and the second data. Instead of the width of the forward scattered light signal waveform and the width of the fluorescence signal waveform, the signal intensity of the forward scattered light signal waveform and the signal intensity of the optical signal waveform may be used, respectively. The microprocessor 20 calculates the third data which represents the area (area hatched in FIG. 6; pulse area of the fluorescence signal) circumscribed by the fluorescence signal waveform (fluorescence signal) and a predetermined baseline relative to the fluorescence signal waveform on the graph (predetermined coordinate plane) shown in FIG. 6. Note that instead of the area of the region surrounded by the fluorescence signal waveform and the predetermined base line, the third data also may be calculated using the height of the fluorescence signal waveform.

Although a configuration in which the microprocessor 20 calculates the first data, the second data, and the third data is shown in the example shown in FIG. 4, the configuration is not limited thereto. For example, the configuration may be such that the first data, the second data, and the third data are output from the signal processing circuit 50 to the microprocessor 20.

### Structure of Cell Information Providing Apparatus 1

The cell information providing apparatus 1 obtains information related to the existence of heterogeneous cells and the trend of increase of heterotypic cells based on results of comparing a first cell number indicating the number of cells having an amount of DNA greater than a threshold value classified into a first group based on the size of the cell and the size of the cell nucleus among the cells contained in the sample, and a second cell number indicating the number of cells having an amount of DNA greater than a threshold value classified into a second group different from the first group on the basis of cell size and cell nucleus size.

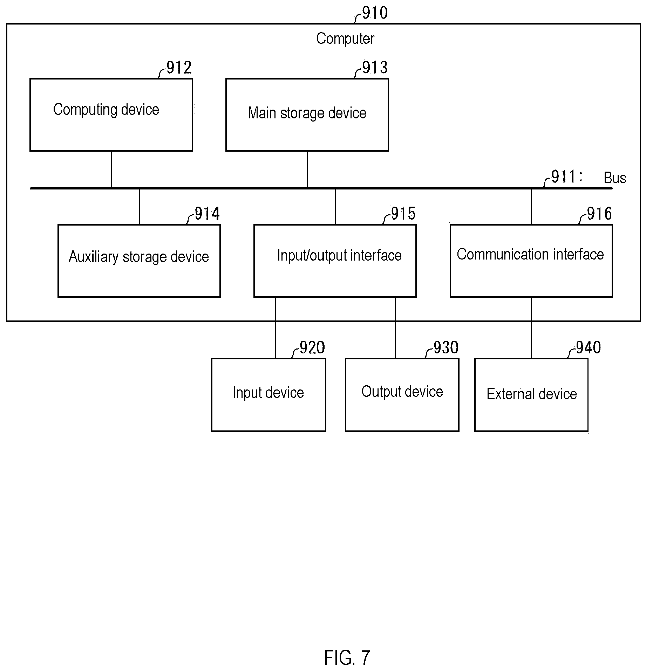

Next, the configuration of the cell information providing apparatus 1 will be described with reference to FIG. 7. FIG. 7 is a block diagram illustrating the structure of a computer 910 usable as the cell information providing apparatus 1.

The computer 910 includes a computing device 912, a main storage device 913, an auxiliary storage device 914, an input/output interface 915, and a communication interface 916 that are connected to each other via a bus 911.

The input/output interface 915 connects an input device 920 for the user to input various information to the computer 910, and an output device 930 for the computer 910 to output various information to the user, and an external device 940 is connected to a communication interface 916. The external device 940 includes, for example, the measuring apparatus 2 and the exam information server 3 shown in FIG. 1.

### Details of Hardware Configuration of Cell Information Providing Apparatus 1

Details of the hardware configuration of the cell information providing apparatus 1 will be described below with reference to FIG. 7. Each block of the cell information providing apparatus 1 may be realized by a logic circuit (hardware) formed in an integrated circuit (IC chip) or the like, or may be realized by software. In the latter case, the cell information providing apparatus 1 can be configured using a computer (electronic computer) as shown in FIG. 7.

FIG. 7 is a block diagram illustrating the structure of a computer 910 usable as the cell information providing apparatus 1. The computer 910 includes a computing device 912, a main storage device 913, an auxiliary storage device 914, an input/output interface 915, and a communication interface 916 that are connected to each other via a bus 911. The arithmetic unit 912, the main storage unit 913, and the auxiliary storage unit 914 also may be, for example, processors (for example, CPU: Central Processing Unit and the like), RAM (random access memory), hard disk drive. The input/output interface 915 connects to an input device 920 for a user to input various information to the computer 910, an output device 930 for the computer 910 to output various kinds of information to the user, and an external device 940 such as the measuring apparatus 2 and the exam information server 3. The input device 920 and the output device 930 may be built in the computer 910 or may be connected (externally attached) to the computer 910. For example, the input device 920 may be a keyboard, a mouse, a touch sensor and the like, and the output device 930 may be a display, a printer, a speaker and the like. An apparatus having functions of both the input device 920 and the output device 930, such as a touch panel in which a touch sensor and a display are integrated, also may be applied. The communication interface 916 is an interface through which the computer 910 communicates with an external device.

Various programs for causing the computer 910 to operate as the cell information providing apparatus 1 are stored in the auxiliary storage device 914. The computing device 912 develops the program stored in the auxiliary storage device 914 on the main storage device 913 and executes a command contained in the program so that the computer 910 functions as each part of the cell information providing device 1. Note that a recording medium for recording information such as a program, which is stored in the auxiliary storage device 914, may be a computer-readable "non-temporary tangible medium", such as a tape, a disk, a card, a semiconductor memory, a programmable logic circuit or the like. The main storage device 913 may be omitted insofar as the computer is capable of executing a program recorded on a recording medium without being developed on the main storage device 913. Note that each of the devices (the computing device 912, the main storage device 913, the auxiliary storage device 914, the input/output interface 915, the communication interface 916, the input device 920, the output device 930, and the external device 940) may be single or may be plural.

The program may be acquired from the outside of the computer 910, in which case the program may be acquired via an arbitrary transmission medium (a communication network, a broadcast wave, or the like). The invention also can also be realized in the form of a data signal embedded in a carrier wave, the program being embodied by electronic transmission.

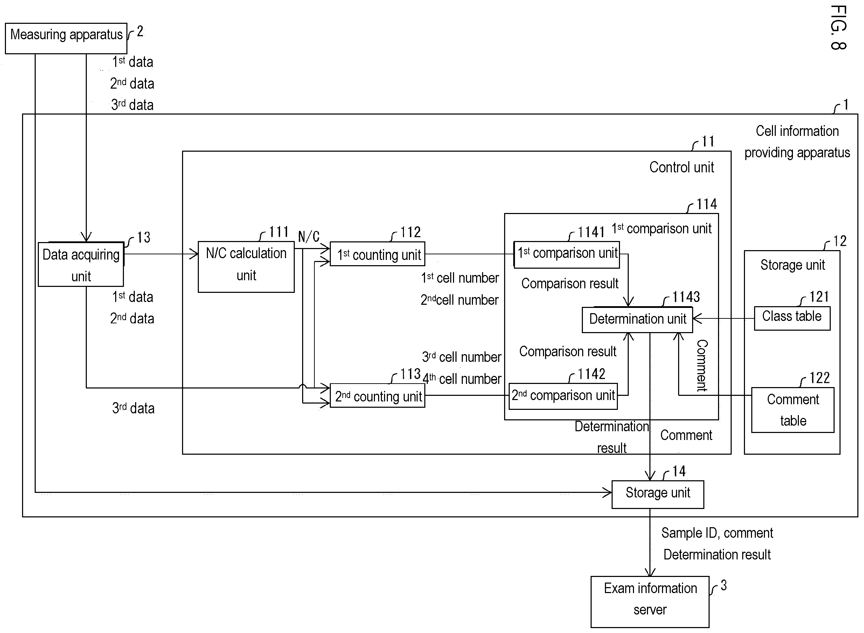

FIG. 8 is a functional block diagram illustrating the software structure of the cell information providing apparatus 1. The cell information providing apparatus 1 includes a data acquiring unit 13, a control unit 11, a storage unit 12 that stores various data used by the control unit 11, and an output unit 14. The control unit 11 controls each unit of the cell information providing apparatus 1. The control unit 11 includes an N/C calculating unit 111, a first counting unit 112 (counting unit), a second counting unit 113, and a dysplasia degree determining unit 114. The dysplasia degree determining unit 114 includes a first comparison unit 1141 (comparison unit), a second comparison unit 1142, and a determination unit 1143. The storage unit 12 stores a classification table 121 and a comment correspondence table 122.

Note that the communication interface 916 shown in FIG. 7 functions as the data acquisition unit 13 and the output unit 14. That is, the cell information providing apparatus 1 receives, via the communication interface 916, first data indicating the size of the cell, second data indicating the size of the cell nucleus, third data indicating the amount of DNA, and sample ID from the optical detection unit such as a flow cytometer. The cell information providing apparatus 1 also outputs the sample ID, the determination result, and the comments to the exam information server 3 via the communication interface 916. The arithmetic unit 912 functions as each functional block of the control unit 11, and the auxiliary storage unit 914 functions as the storage unit 12.

### Processing Flow Performed by Cell Information Providing Apparatus

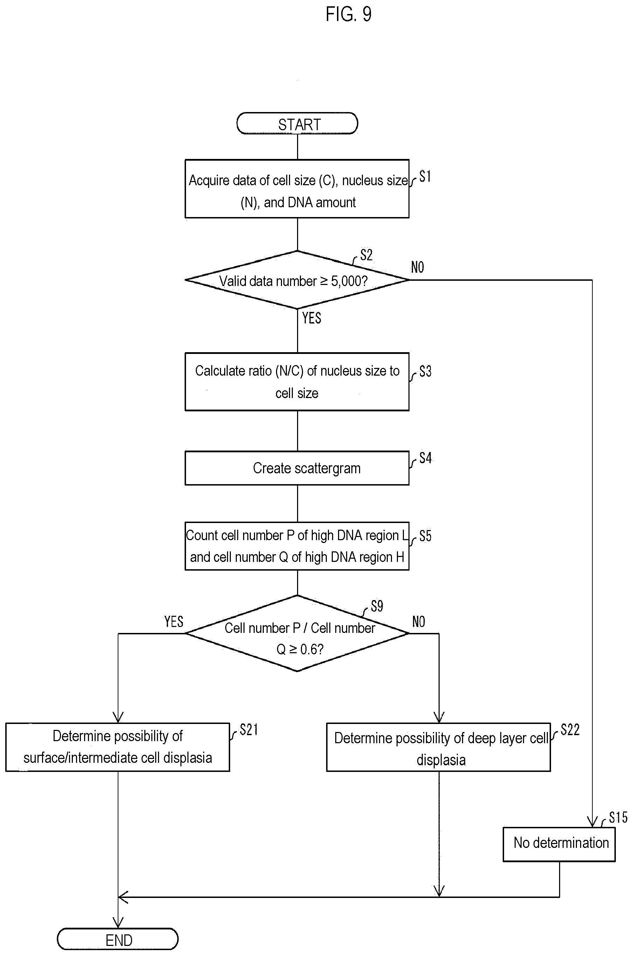

The flow of processing performed by the cell information providing apparatus 1 will be described below with reference to FIG. 9 while referring to function blocks shown in FIG. 8. FIG. 9 is a flowchart showing an example of the processing flow performed by the cell information providing apparatus 1.

The data acquiring unit 13 acquires from the measuring device 2 the first data indicating the size of the cell, the second data indicating the size of the cell nucleus, and the third data indicating the amount of DNA for the cells collected from the epithelial tissue of the subject (step S1: data acquiring step). Note that the data acquiring unit 13 is configured to acquire from the measuring device 2 measurement data such as forward scattered light signal waveform data obtained from individual cells contained in the analysis sample (sample), fluorescence signal waveform data of each cell. In this case, the cell information providing apparatus 1 further includes a data calculation unit (not shown) for calculating the first data, the second data, and the third data by using the forward scattered light signal waveform data and the fluorescence signal waveform data. Note that when an individual sample ID (identification information) is assigned to each analysis sample including cells collected from each subject or tissue of the subject, the data acquiring unit 13 that performs the above process is configured to acquire the sample ID together with the measurement data.

The data acquiring unit 13 counts the number of valid data from which data indicating a size that can not be a cell has been removed and the like. When the number of valid data is less than a target value (for example, 5000) set as the number of measurement data necessary for the cell information providing apparatus 1 to output a determination result that a certain reliability is ensured (in step S2: NO), the output unit 14 outputs information that the number of valid data is insufficient and it is impossible to make a determination (step S15). Note that the above-mentioned target value of "5000" is a number commonly used as an index for determining the suitability of cytological diagnostic examinations, and also in this embodiment is It is adopted as a target value for guaranteeing the accuracy of analysis.

When the number of valid data is equal to or larger than the target value (YES in step S2), the N/C calculating unit 111 calculates the ratio (N/C ratio) between the cell size and the cell nucleus size of each cell (Step S3).

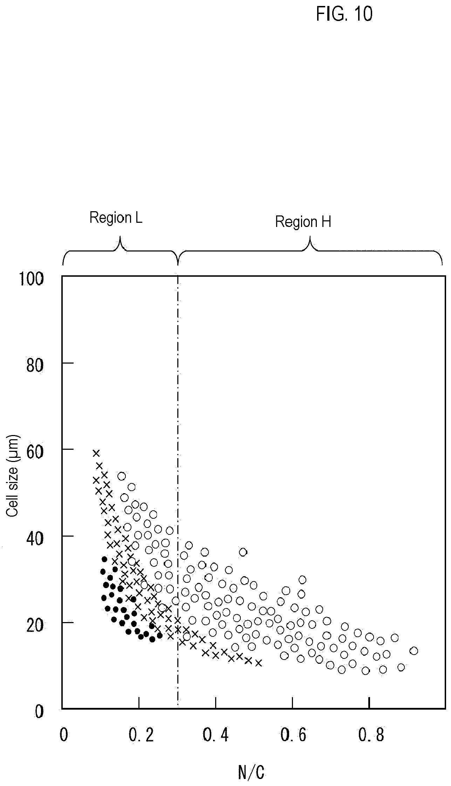

The N/C calculating unit 111 also creates a scattergram such as shown in FIG. 10 (step S4). The created scattergram may be output to the display device (not shown) from the output unit 14 together with the determination result and displayed.

That is, the N/C calculating unit 111 calculates the ratio between the size of the cell and the size of the cell nucleus for each cell as a characteristic value regarding the morphology of the cell calculated from the first data and the second data. Below, the case where the N/C calculating unit 111 calculates the ratio (hereinafter referred to as "N/C ratio") between the cell size (C) and the cell nucleus size (N). However, the present invention is not limited to this inasmuch as, for example, the value calculated by the N/C calculating unit 111 may be a value related to the form of the cell. That is, the N/C calculating unit 111 may calculate the cell size (C/N ratio) with respect to the cell nucleus size as a characteristic value. Alternatively, the N/C calculating unit 111 may calculate a value indicating the morphology of each cell from the relationship between the cell nucleus and the size of the cell using a predetermined relational expression. Note that when a characteristic value different from the N/C ratio is adopted, the second threshold value described later can be appropriately set according to the adopted characteristic value.

The first counting unit 112 acquires the N/C ratio of each cell and the DNA amount of each cell. The first counting unit 112 sets a second threshold value (for example, N/C 0.3). For example, in FIG. 10, the second threshold value is indicated by an alternating long and short dash line. The first counting unit 112 counts the number of cells P (first cell number) in which the DNA amount is large in a region where the N/C ratio is equal to or lower than the second threshold value (region L in FIG. 10), and the cell number Q (second cell number) having a large DNA amount in the region H of FIG. 4) (step S5: first counting step (counting step)). That is, the cell number P is the number of cells having a DNA amount larger than the first threshold value (threshold) among the cells belonging to the region L (classified into the first group) based on the size of the cell and the size of the cell nucleus. On the other hand, the cell number Q is the number of cells having a DNA amount higher than the first threshold value among cells belonging to the region H (classified into the second group) based on the size of the cell and the size of the cell nucleus.

For example, the first counting unit 112 respectively counts the cell number P indicating the number of cells whose third data value is greater than the first threshold among the cells having an N/C value equal to or less than the second threshold value and the cell number Q indicating the number of cells whose third data value is greater than the first threshold value among cells whose N/C ratio is greater than the second threshold value using the N/C ratio calculated by the N/C calculating unit 111, and third data indicating the amount of DNA of the cell obtained from the data acquiring unit 13. That is, a cell having a relatively small N/C ratio and a cell having a relatively large N/C ratio are distinguished by using the second threshold value set for the N/C ratio. Note that the second threshold value is a value obtained by distinguishing between the surface/intermediate layer cells (cells on the surface layer side) including the cells of the surface layer and the intermediate layer and the deep layer cells including the parabasal layer and the cells of the basal layer (cells on the basal layer). Note that although a case where one value is used as the second threshold value will be described as an example, the present invention is not limited to this case, and the second threshold value may be a value having a constant width. For example, a threshold value defining an upper limit for defining a cell having a relatively small N/C ratio and a threshold value defining a lower limit for defining a cell having a relatively large N/C ratio may be different values.

Note that the first counting unit 112 is configured to create a scattergram for the first data and the N/C ratio of each cell, for example, and count the number of cells having a data set to be plotted within a predetermined area (area L and the area H in FIG. 10). In this case, the second threshold value is set as a boundary line to divide two regions having different ranges of the N/C ratio of the scattergram. The first counting unit 112 counts the number of cells contained in each of the two areas.

On the other hand, the "first threshold value" is a value set between a value indicating the amount of DNA of a diploid cell and a value indicating a DNA amount larger than that of a diploid cell, and is a value that is set to distinguish between cells having a DNA amount corresponding to diploid cells and cells having a DNA amount greater than diploid cells.

That is, the first counting unit 112 respectively counts the number of cells having a DNA amount higher than the first threshold value among the cells having an N/C ratio equal to or lower than the second threshold value, and the number of cells having a DNA amount higher than the first threshold value among cells having an N/C ratio higher than the second threshold value.

Note that the first counting unit 112 also may generate a histogram of the DNA amount of the cell, for example, and the first threshold value may be calculated from the mathematical mode and half width of the peak corresponding to the diploid cells in the histogram using a statistical method.

### Method of Counting the First Cell Number and Second Cell Number in Step S5

A method of counting the first cell number and the second cell number by the first counting unit 112 will be described below with reference to FIG. 10. FIG. 10 is a schematic diagram illustrating the number of cells counted by the first counting unit 112. In the scattergram with the N/C ratio on the horizontal axis and the first data on the vertical axis shown in FIG. 10, each plot corresponding to the data of the cells in the measurement sample has a distribution exhibiting a crescent shape which is sloping downward.

In step S5, the first counting unit 112 counts cells having a DNA amount larger than the first threshold value based on the pulse area of the fluorescence signal obtained from the cell nucleus of each cell, that is, the fluorescence amount. Specifically, the first counting unit 112 acquires the N/C ratio of each cell calculated by the N/C calculating unit 111, and the DNA amount of each cell corresponding to the fluorescence amount obtained from the cell nucleus of each cell. Then, the first counting unit 112 counts the number of cells P in which the DNA amount in the area L where the N/C ratio is equal to or less than the second threshold value (for example 0.3), and the number of cells Q in the area H in which the DNA amount is larger than the second threshold value.

Note that FIG. 10 is a schematic diagram wherein plots corresponding to cells having a DNA amount equivalent to diploid cells are for indicated by "x" for convenience, plots corresponding to cells having a smaller DNA amount than diploid cells are indicated by solid black circles, and plots corresponding to cells having a larger amount of DNA than diploid cells are indicated by "open white circles". The number of cells P counted by the first counting unit 112 is the number of white circles in the area L in FIG. 10, and the cell number Q is the number of white circles in the area H.

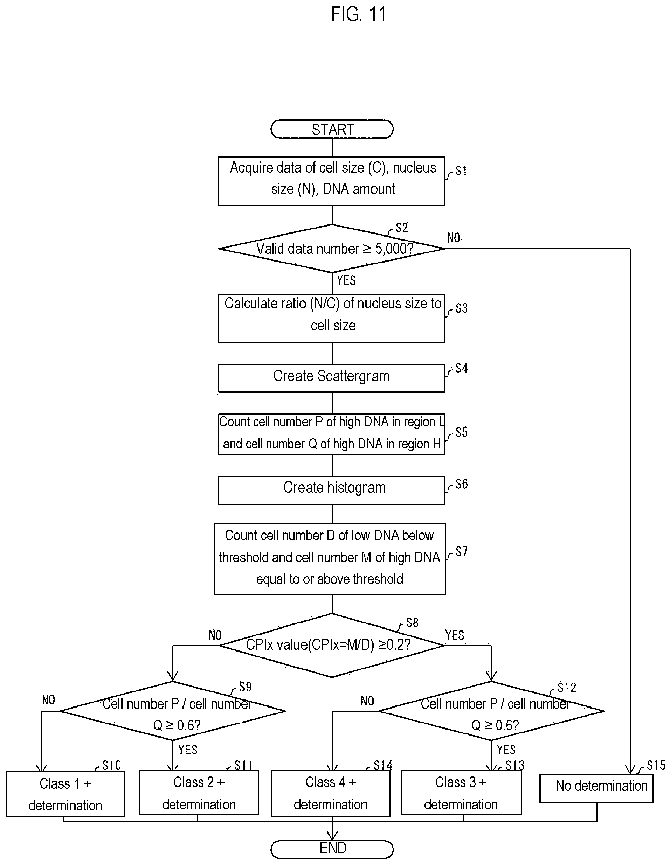

Returning to the flowchart of FIG. 11, the first comparison unit 1141 compares the cell number P counted by the first counting unit 112 with the cell number Q. The determination unit 1143 determines the state of the cell based on the comparison result. For example, if the result of comparison is cell number P/cell number Q ≧ 0.6 (YES in Step S9: comparison step), the determination unit 1143 determines that there is a possibility that findings related to dysplasia may appear in the surface layer and middle layer of the epithelial tissue (Step S21: determination step). [fuzzy]For example, if the result of comparison is cell number P/cell number Q ≧ 0.6 (YES in Step S9: comparison step), the determination unit 1143 determines that there is a possibility that findings related to dysplasia may appear in the surface layer and middle layer of the epithelial tissue (Step S21: determination step). Note that at least one of the comparison results as described above and the determination result including the information on the state of the tissue containing the cells in the sample is output from the output unit 14 to an external device such as a display device (see FIG. 7) (output step).

### Processing Flow Performed by Cell Information Providing Apparatus

The control unit 11 includes the second counting unit 113, and the dysplasia degree determining unit 114 includes the second comparison unit 1142; the flow of a process in which the determination unit 1143 makes a determination using the ratio of the number of cells having a DNA amount equal to or less than the first threshold value to the number of cells having a DNA amount greater than the first threshold value (CPIx value) will be described below with reference to FIG. 11. FIG. 11 is a flowchart showing another example of the processing flow performed by the cell information providing apparatus 1. Note that, for convenience of explanation, the same reference numerals are attached to the steps that perform the same processing as the steps described in the above embodiment, and description thereof is omitted.

Using the N/C ratio calculated by the N/C calculating unit 111 in step S3, the second counting unit 113 specifies the measurement data of the cells on the basal side, and uses the data of the amount of DNA contained in the specified measurement data to create a histogram (step S6). Specification of measurement data of cells on the basal side is performed by extracting measurement data of cells having an N/C ratio of 0.3 or more, for example. Then, in the histogram, the second counting unit 113 counts the number of cells D (third cell number) having a DNA amount equal to or lower than the first threshold value, and the cell number M (fourth cell number) having a DNA amount larger than the first threshold value, (step S7: second counting step).

### Method of Counting Cell Number D and Cell Number M in Step S8

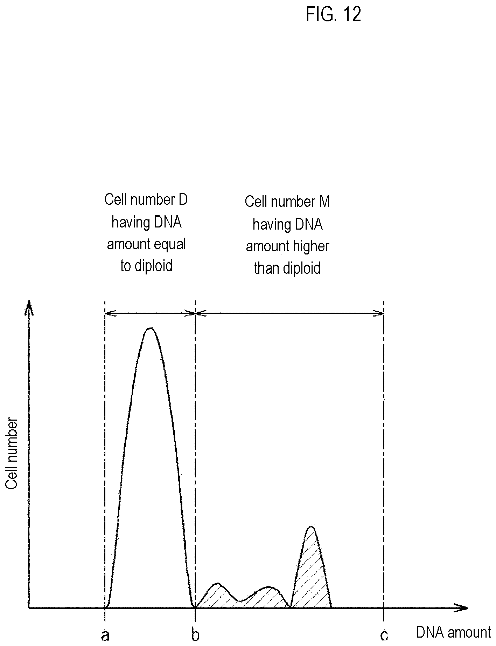

A method of counting the number of cells D and the number of cells M by the second counting unit 113 will be described below with reference to FIG. 12.

In FIG. 12, the vertical axis represents the number of cells and the horizontal axis represents the DNA amount of the cells. Cells belonging to the range of a to b shown in FIG. 12 are cells having a DNA amount (see "2C" in FIG. 3) corresponding to the DNA amount of diploid cells. The second counting unit 113 counts the number of cells belonging to the range of a to b shown in FIG. 12 as the cell number D. On the other hand, cells belonging to the range larger than b and smaller than c are cells having a larger amount of DNA ("4C" in FIG. 3) than the DNA amount of diploid cells. The second counting unit 113 counts the number of cells belonging to the range larger than b and lower than c shown in FIG. 12 as the cell number M.

That is, cells counted as the cell number D include normal cells in the G0 and G1 phases, and cells counted as the cell number M include cells in the S phase, G2 phase, and M phase, and cancerous cells and heterogeneous cells.

Note that the value of b (first threshold value) shown in FIG. 12 may be a value that is set in order to distinguish whether the cell has a DNA amount larger than that of a diploid cell, for example, or may be a value preset based on the amount of DNA possessed by a diploid cell. For example, considering the accuracy and reliability of analysis, the range of the normal amount of DNA (between a and b in FIG. 12) and the range of abnormal DNA amount (between b and c in FIG. 12) in the cells to be analyzed also can be selected by various experiments, inspection and the like.

Returning to the flowchart of FIG. 11, the processing flow performed by the cell information providing apparatus 1 will continue below. Note that in the following description the second comparison unit 1142 compares the CPIx value with a predetermined reference value of 0.2. The second comparison unit 1142 calculates (step S8) the CPIx value (cell number M/cell number D) using the cell number D and the cell number M calculated by the second counting unit 113 (step S7), and compares CPIx value to 0.2. On the other hand, the first comparison unit 1141 performs the same processing as the above-described step S9 (step S9 and step S12: comparison step).

The determination unit 1143 refers to the classification table 121 and determines as follows.
- When the CPIx value is less than 0.2(NO in step S8) and the cell number P/cell number Q is less than 0.6(NO in step S9), classification 1 is determined (step S10).
- When the CPIx value is less than 0.2(NO in step S8) and the cell number P/cell number Q is 0.6or more (YES in step S9), classification 2 is determined (step S11).
- When the CPIx value is0.2 or more(YES in step S8) and the cell number P/cell number Q is0.6 or more (YES in step S12), classification 3 is determined (step S13).
- When the CPIx value is0.2 or more(YES in step S8) and the cell number P/cell number Q is less than 0.6(NO in step S12), classification 4 is determined (step S14).

### Details of Classification Table 121

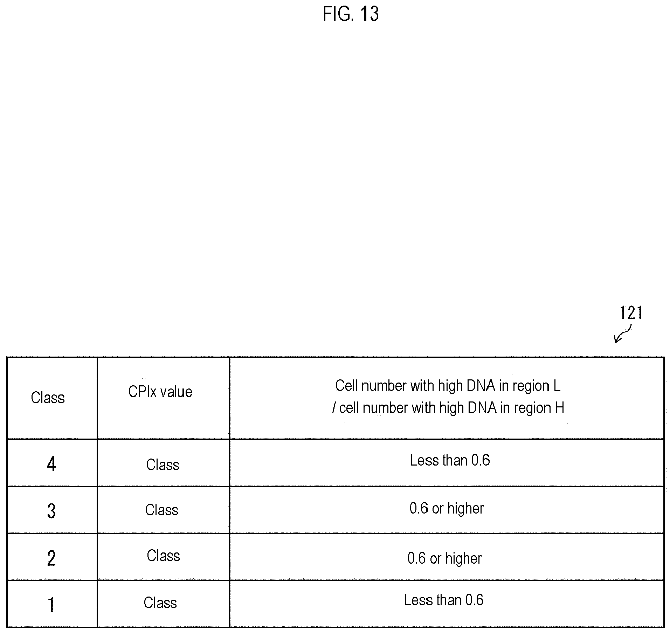

Classification table 121 will be described below with reference to FIG. 13. The classification table 121 shown in FIG. 13 defines an example of a first criterion and a second criterion referred to when the determination unit 1143 determines the degree of dysplasia of cells included in an analysis sample. The column of "CPIx value" in the classification table shown in FIG. 13 is a standard concerning the second criterion, and the column "number of cells with a large amount of DNA in region L/number of cells with a large amount of DNA in region H" (see FIG. 10) is defined in relation to the first criterion.

The CPIx value is a value calculated as the ratio of the cell number D to the cell number M, and expresses the ratio of cells having a DNA amount larger than the DNA amount of diploid cells. When the CPIx value is high, it can be utilized as a value indicating the degree of suspected existence of cancerous cells and heterogeneous cells.

Although whether the ratio of "number of cells having a large amount of DNA in region L" to "number of cells with a large amount of DNA in region H" is 0.6 or more is the criterion in the example shown in FIG. 13, the invention is not limited to this. For example, whether the ratio of "number of cells having a large amount of DNA in region L" to "number of cells with a large amount of DNA in region H" is 1.0 or more also may be set as a criterion. Similarly, although the CPIx value is0.2or more in the example shown in FIG. 13, it is not limited to this.

For example, the determination unit 1143 may make a determination according to the following classifications.
- (Classification 1) When the ratio of cell number D to cell number M is less than 0.2, and the ratio of the cell number P to the cell number Q is less than 0.6, no finding is determined.
- (Classification 2) When the ratio of cell number D to cell number M is less than 0.2, and the ratio of the cell number P to the cell number Q is 0.6or more, it is determined that there is a possibility that dysplasia will be observed in the surface layer and the intermediate layer of epithelial tissue.
- (Classification 3) When the ratio of cell number D to cell number M is0.2or more and the ratio of the cell number P to the cell number Q is 0.6 or more, it is determined that there is a high possibility that dysplasia will be observed in the surface layer and the intermediate layer of epithelial tissue.
- (Classification 4) When the ratio of cell number D to cell number M is0.2or more and the ratio of the cell number P to the cell number Q is less than 0.6, it is determined that there is a high possibility that dysplasia will be observed in the parabasal layer and the basal layer of epithelial tissue.

Note that the determination result as described above may be output from the output unit 14 to an external device (not shown) such as a display device. The output unit 14 also may output a comment corresponding to the determination result together with the determination result, based on a comment correspondence table 122.

The processing flow shown in FIG. 11 is merely an example, and the present invention is not limited to this example. For example, the processing of step S3 to step S5 and the processing of step S6 to step S7 may be performed in parallel, or the processing of step S6 to step S7 may be performed before the processing of step S3 to step S5. Similarly, the sequence of the processing in step S8 and the processing in step S9 and step S12 may be reversed. That is, the process of step S8 may be performed for each of the cases of YES and NO in the process of step S9.

### Details of Comment Correspondence Table 122

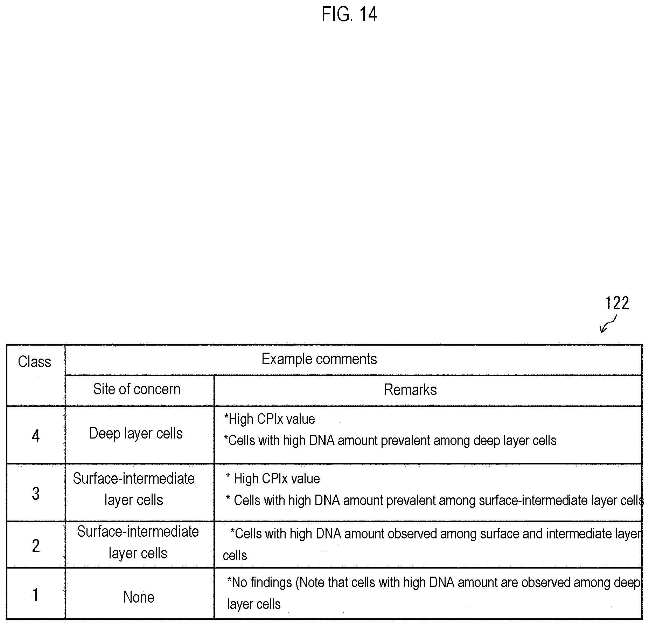

The determination unit 1143 also may be configured to refer to the comment correspondence table 122 shown in FIG. 14 and select a comment corresponding to the determination result (for example, the classifications 1 to 4) (not shown). As shown in FIG. 14 in the comment correspondence table 122, information on the area of concern and remarks are associated with each category. Remarks may include information on the determination result, information on various values calculated using the cell number P, the cell number Q, the cell number D, and the cell number M and the like. For example, in the remarks corresponding to Classification 2 in FIG. 14, "cells observed with a large amount of DNA among the surface/intermediate layer cells" is a comment related to the determination result. On the other hand, when classification 1 is determined, for example, the site of concern is set to "none", and the remark related to the determination is "there is no finding" (cells having a large amount of DNA are observed among deep layer cells)". As shown in the remarks corresponding to Classification 3 and Classification 4 of FIG. 14, information based on comparison of the cell number D and the cell number M, that is, "CPIx value is high", also may be included.

The output unit 14 associates the sample ID assigned to each sample with the determination result and the comment acquired from the determination unit 1143, and outputs the result to an external device (not shown) such as a display device (output step).

Specifically, in addition to the determination result obtained from the determination unit 1143, the output unit 14 outputs at least one of the result of comparing the cell number P with the cell number Q, and the result of comparing the cell number D with the cell number M. Note that the external device may be a personal computer (not shown) used by a physician, a nurse, or the like, as long as it has a function of presenting a determination result to the user, for example, a microscopy terminal apparatus 4 used by a cytodiagnostician or the like. Alternatively, the cell information providing apparatus 1 may include a display device. The output unit 14 also may be configured to output at least one of the scattergram created by the first counting unit 112 and the histogram created by the second counting unit 113 in association with the determination result and comment.

Note that although the output unit 14 acquires the sample ID from the measuring device 2 in FIG. 6, the configuration is not limited thereto. For example, the data acquiring unit 13 may acquire the sample ID together with various data, and the output unit 14 may acquire the sample ID via the data acquiring unit 13.

Note that the cell information providing apparatus 1 may be connected to a plurality of measuring apparatuses 2 via a network such as a local area network (LAN), and acquire measurement data from a plurality of measuring apparatuses 2. Alternatively, the measuring apparatus 2 and the cell information providing apparatus 1 may be integrated.

### Relationship Between Classifications 1 to 4 and Determination Results of Histological Diagnosis and Cytodiagnosis

How the determination results "classification 1" to "classification 4" output from the cell information providing apparatus 1 correspond to the classification in the tissue diagnosis and the cytological diagnosis is explained below using FIG. 15. FIG. 15 is a diagram for illustrating the relationship between precancerous lesions in epithelial tissue and classification in cell diagnosis.

For example, in the histology of the cervix, the process from the normal state to cancer is classified into several stages including "Normal", "CIN1", "CIN2", "CIN3", and "Cancer". The five schematic diagrams shown at the top of FIG. 15 show the states of the cells at these five stages. Among them, the stage called "cervical intraepithelial neoplasia (CIN)" corresponds to the stages of "CIN1", "CIN2", and "CIN3".

"CIN1" is a state in which abnormal cells are proliferating in one-third of the area from the basal layer to the surface layer, and there is a high possibility that spontaneous regression will occur. Therefore, it is determined that treatment is unnecessary in "CIN1". FIG. 15 shows a state in which abnormal cells are observed in the parabasal layer, and cells in which cell change due to HPV proliferation is observed in the surface layer and intermediate layer cells. "CIN2" is a state in which abnormal cells are proliferating in two-thirds of the area from the basal layer toward the surface layer, and it is judged that treatment is necessary in "CIN2". "CIN3" is a state in which abnormal cells are proliferating almost throughout the basal layer toward the surface layer, and it is determined that treatment is necessary in "CIN3". It is desirable to be able to detect a risk of cancer at an early stage of cancer such as "CIN2" to "CIN3" before the cancer progresses to "Cancer" in order to start treatment of cancer early.

On the other hand, in the case of cervical cancer screening, cytological examination is performed first. The classification method referred to as Bethesda classification is applied to the result of this inspection. The Bethesda classification uses multiple stages including Normal (NILM), "Low SIL (LSIL)" in which mild abnormality is presumed, "High SIL (HSIL)" in which high abnormality is presumed, and "SCC" in which uterine cancer is suspected. Note that "SIL" is an abbreviation for the English term meaning squamous intraepithelial lesion. "CIN1", "CIN2", and "CIN3" in the histological diagnosis, and "LSIL" and "HSIL" in Bethesda classification are related as shown in FIG. 15.
"Classification 1" of the determination result output from the cell information providing apparatus 1 corresponds to the stage of "normal" of the histological diagnosis and the stage of "normal (NILM)" of cytodiagnosis.
"Classification 2" corresponds to a stage closer to normal among the "CIN1" of the histological diagnosis, and corresponds to the stage of "LSIL" of cytodiagnosis.
"Classification 3" corresponds to the stage in "CIN2" from "CIN1" to "CIN2" close to "CIN3" of histological diagnosis, and corresponds to the stage from "LSIL" to the stage antagonistic to "LSIL' and "HSIL" of cytodiagnosis.
"Classification 4" corresponds to the stage from "CIN2" near "CIN 3" up to the stage of "Cancer" of the histological diagnosis, and from the stage antagonistic to "LASIL" and "HSIL" up to "SCC" of cytodiagnosis.

### Exam Information Server 3

The exam information server 3 is a storage device that stores the determination result output by the cell information providing apparatus 1, comments and the like in association with the sample ID. The exam information server 3 stores an image obtained by imaging the smear sample in cytodiagnosis, and findings input by a cell examiner who performed cytodiagnosis in association with the sample ID.

A report is prepared based on the information stored in the exam information server 3, and the report is delivered (provided) from the laboratory facility 120 to the medical facility 110.

### Structure of Microscopy Terminal Apparatus 4

The configuration of the microscopy terminal apparatus 4 will be described below with reference to FIG. 1. The microscopy terminal apparatus 4 is provided with a control unit 41 which comprehensively controls each function of the microscopy terminal apparatus 4, and a display unit 42 which displays images and the like. The control unit 41 also includes an image processing unit 411, a sample ID acquiring unit 412, and an input unit 413.

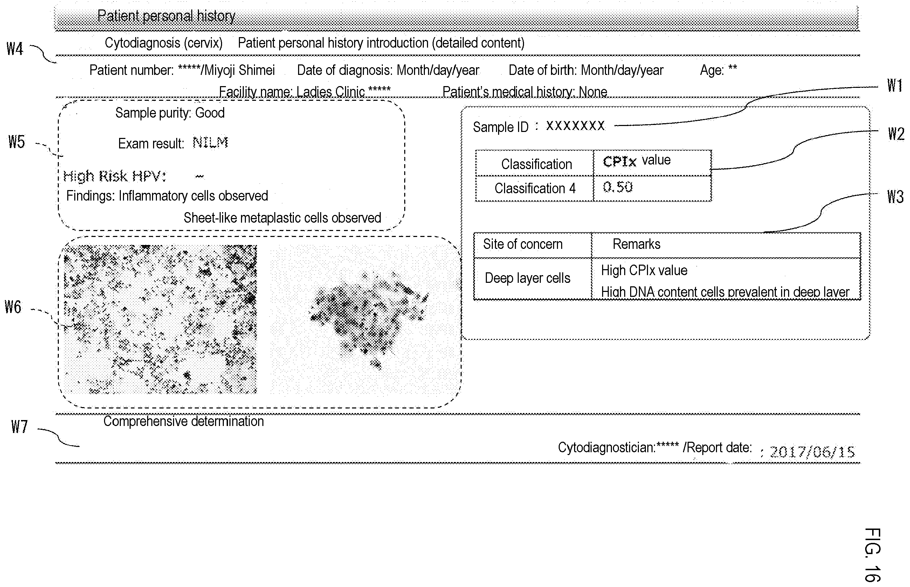

The image processing unit 411 generates a screen display combining the image captured by the imaging device 5, the examination finding input by the cytodiagnostician, the determination result read from the exam information server 3, and displays it on the display unit 42 (see FIG. 16).

The sample ID acquiring unit 412 acquires the sample ID assigned to the smear sample. The acquired sample ID is used for reading the determination result and comment related to the cell sample from the exam information server 3, and recording the image of the smear sample and the findings of the cytodiagnosis and the like in the exam information server 3.

The input unit 413 acquires the examination finding input by cytodiagnostician who performed cytological diagnosis.

### Cell Information Provided by Cell Information Providing System 100

A case where the determination result by the cell information providing apparatus is used by the microscopy terminal apparatus 4 in the cell information providing system 100 is described below as an example with reference to FIG. 16. FIG. 16 is a diagram showing an example of a screen display on which the microscopy terminal apparatus displays the microscope inspection image and the determination result by the cell information providing apparatus.

In the column W4, "patient number", "reception date", "birth date", "age", "facility name", and "past history" are described. "Facility name" is the name of the medical facility 110, and is a medical institution from which a cell sample is collected from a patient. "Patient number" can be used as a sample ID, but a sample ID may be given for each cell sample or for each patient. In this case, the sample ID is associated with the patient number. In the example shown in FIG. 16, "xxxxxx" in column W1 is a sample ID assigned to each cell sample.

In the column W2, the CPIx value 0.50 provided from the cell information providing apparatus 1 and determination result "classification 4" are displayed, and in the column W3, comments (see FIG. 14) corresponding to the determination result "classification 4" are displayed.

In column W5, "sample preparation accuracy", "exam result", and findings input by a cytodiagnostician who conducted a microscopic examination are displayed. In column W6, the image captured during the microscopic examination is displayed.

In column W7, there is provided a column for entering "comprehensive determination", for example, in the stage of preparing the report, and the determination result input as the ultimate determination is displayed.

The present invention is not limited to the above-described embodiments, and various modifications are possible within the scope indicated in the claims, and embodiments obtained by appropriately combining technical means respectively disclosed in different embodiments are also included in the technical scope of the present invention. Example

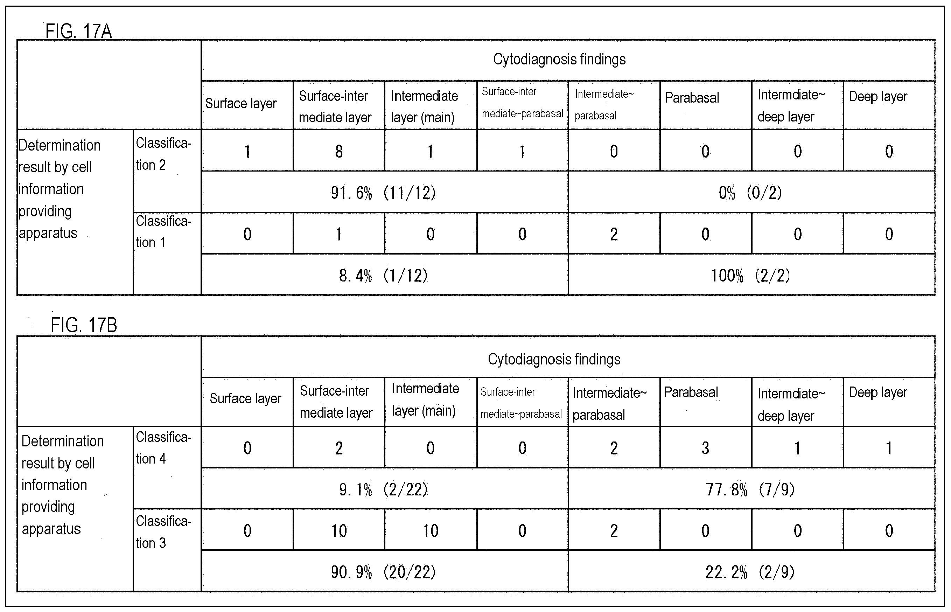

An example in which the determination result by the cell information providing apparatus 1 of the present invention is verified will be described below with reference to FIG. 17A and FIG. 17B. FIG. 17A and FIG. 17B show the results in one example of verifying the determination result by the cell information providing apparatus 1.

FIG. 17A shows results when the CPIx value is less than 0.2, and the determination was made by the cell information providing apparatus 1 on 14 samples having findings related to the appearance location of heterogeneous cells in cytodiagnosis.

The cell information providing apparatus 1 succeeded in determining 11 samples out of the 12 samples having findings concerning dysplasia of the surface layer and the intermediate layer cells in cytodiagnosis as "classification 2", and the accuracy of the determination was 91.6%.

FIG. 17B shows results when the CPIx value is 0.2or more, and 45 samples having findings concerning the location of heterogeneous cells in cytodiagnosis were determined by the cell information providing apparatus 1.

The cell information providing apparatus 1 succeeded in determining 20 samples out of 31 samples having findings concerning dysplasia of the surface layer and intermediate layer cells in cytodiagnosis as "class 3", and the accuracy of the determination was90.9%. The cell information providing apparatus 1 also succeeded in determining 7 out of 9 samples having findings concerning dysplasia from the basal layer to the intermediate layer cells in "cytodiagnosis" as "class 4", and the accuracy was77.8%.

As described above, the cell information providing apparatus 1 was confirmed to be capable of determining dysplasia of cells contained in a sample with high accuracy.

## Claims

1. A cell analysis method comprising:
a counting step of counting, among cells included in a sample, a first cell number indicating the number of cells classified into a first group based on cell size and cell nucleus size and having a DNA amount greater than a threshold value, and a second cell number indicating the number of cells classified into a second group that is different from the first group based on the cell size and cell nucleus size and having a DNA amount greater than the threshold value; and
a comparing step of comparing the first cell number with the second cell number.

2. The cell analysis method according to claim 1, wherein
the counting step comprises classifying the cells included in the sample based on the cell size and the cell nucleus size so that cells on the surface layer side are included in the first group and cells on the basal side are included in the second group.

3. The cell analyzing method according to claim 1 or 2, wherein
the counting step comprises classifying the cells included in the sample into the first group and the second group based on a ratio between the cell size and the cell nucleus size.

4. The cell analysis method according to claim 3, wherein
the counting step comprises classifying the cells included in the sample into the first group and the second group by comparing the ratio with a second threshold value.

5. The cell analysis method according to any one of claims 1 to 4, wherein
the counting step comprises counting cells having a DNA amount greater than the threshold value based on an amount of fluorescence obtained from the cell nucleus.

6. The cell analysis method according to any one of claims 1 to 5, wherein
the threshold value is a value set between a value indicating a DNA amount of a diploid cell and a value indicating a DNA amount larger than the diploid cell.

7. The cell analysis method according to any one of claims 1 to 6, wherein
the cells contained in the sample are cells detached from tissue.

8. The cell analysis method according to claim 7, wherein
the tissue is epithelial tissue.

9. The cell analysis method according to any one of claims 1 to 8, further comprising:
an outputting step of outputting information on a state of a tissue including the cells contained in the sample based on the comparison result in the comparing step.

10. The cell analysis method according to claim 9, wherein
the outputting step comprises outputting information on a presence or absence of dysplasia in the surface layer and an intermediate layer of the tissue depending on whether the first cell number is larger than the second cell number.

11. The cell analysis method according to claim 9 or 10, further comprising:
a second counting step of counting, among cells contained in the sample, a third cell number indicating the number of cells having a DNA amount equal to or lower than the threshold value, and a fourth cell number indicating the number of cells having a DNA amount greater than the threshold value;
wherein the outputting step comprises outputting the state of the tissue based on a comparison result between the first cell number and the second cell number and a comparison result between the third cell number and the fourth cell number.

12. A cell information providing apparatus comprising:
a counting unit configured to count, among cells included in a sample, a first cell number indicating the number of cells classified into a first group based on cell size and cell nucleus size and having a DNA amount greater than a threshold value, and a second cell number indicating the number of cells classified into a second group that is different from the first group based on the cell size and cell nucleus size and having a DNA amount greater than the threshold value; and
a comparing unit configured to compare the first cell number with the second cell number.

13. The cell information providing apparatus according to claim 12, further comprising:
a second counting unit configured to count, among the cells included in the sample, a third cell number indicating the number of cells having a DNA amount equal to or lower than the threshold value, and a fourth cell number indicating the number of cells having a DNA amount greater than the threshold value; and
a determining unit configured to determine a state of a tissue based on a comparison result between the first cell number and the second cell number and a comparison result between the third cell number and the fourth cell number.

14. A cell information providing system comprising:
the cell information providing apparatus of claim 12 or 13;
a storage device configured to store cell information provided from the cell information providing apparatus in association with identification information of the sample; and
a microscopy terminal apparatus configured to acquire an image obtained by imaging the sample to which the identification information is attached, read out the cell information corresponding to the identification information from the storage device, and display the acquired image and the cell information read out from the storage device.

15. A computer-readable recording medium storing a computer program which causes a computer to perform:
counting, among cells included in a sample, a first cell number indicating the number of cells classified into a first group based on cell size and cell nucleus size and having a DNA amount greater than a threshold value, and a second cell number indicating the number of cells classified into a second group that is different from the first group based on the cell size and cell nucleus size and having a DNA amount greater than the threshold value; and
comparing the first cell number with the second cell number.
